Europäisches Patentamt

European Patent Office

Office européen des brevets

(11) Publication number: **0 244 999 B1**

# EUROPEAN PATENT SPECIFICATION

(45) Date of publication of patent specification: **21.10.92**    (51) Int. Cl.[5]: **C12M 1/12**

(21) Application number: **87303694.1**

(22) Date of filing: **27.04.87**

(54) **Use of charged particles in the membrane filtration of liquid cell culture media.**

(30) Priority: **28.04.86 US 854981**
         **10.04.87 US 34657**

(43) Date of publication of application:
      **11.11.87 Bulletin 87/46**

(45) Publication of the grant of the patent:
      **21.10.92 Bulletin 92/43**

(84) Designated Contracting States:
      **AT BE CH DE ES FR GB IT LI NL SE**

(56) References cited:
      **EP-A- 0 009 394**
      **EP-A- 0 009 395**
      **EP-A- 0 035 121**
      **US-A- 4 036 693**

(73) Proprietor: **ROHM AND HAAS COMPANY**
      **Independence Mall West**
      **Philadelphia Pennsylvania 19105(US)**

(72) Inventor: **Cabral, Joaquim Manuel Sampio**
      **Rue Vila Catio, lote 397 1 F**
      **1800 Lisboa(PT)**
      Inventor: **Cooney, Charles Leland**
      **35 Chestnut Place**
      **Brookline, Massachusetts 02146(US)**
      Inventor: **Robinson, Elizabeth Marie**
      **929 Mass Avenue, No 6E Cambridge**
      **Massachusetts 02139(US)**

(74) Representative: **Tanner, James Percival et al**
      **ROHM AND HAAS (UK) LTD. European Oper-**
      **ations Patent Department Lennig House 2**
      **Mason's Avenue**
      **Croydon CR9 3NB(GB)**

## Description

The present invention is concerned with a method for filtering liquid cell culture media, such as fermentation broths, using charged particles, e.g. ion exchange resins, filtering aids.

The use of ion exchange resins as filtering aids in the membrane filtration of liquid cell culture media such as fermentation liquors is known. US-A-4,200,695 to Chong et al describes flocs prepared by mixing microsized cation and anion exchange resins and the use of these preformed flocs as filtering agents for fermentation broths, and suggests that cation or anion exchange resins individually may be used in ultrafiltration within the lumens of fine hollow fibers (col. 9, line 59 to col. 10, line 44). In another context (col. 12, lines 17-30), US-A-4,200,695 describes preparation of the flocs, in the liquid to be treated and filtered, by adding emulsions of the cationic and anionic emulsion copolymer resins to the liquid.

Combinations of fine particle size anion and cation exchange resins have also been used sequentially for other purposes, such as the removal, from potable water, of humic substances which react with chlorine to produce toxic trihalomethanes (as described in US-A-4,537,683 to Isacoff and Neely), or the simultaneous decolorization and clarification of impure sugar solutions (as described by Cartier, US-A-4,247,340).

We have now surprisingly found that if first microsized, charged, particulate material, e.g. ion exchange resin, is mixed with a liquid cell culture medium and the resulting suspensions are membrane filtered, several highly significant advantages are obtained when compared to membrane filtration of the cell culture medium in the absence of such charged particulate material. These and other advantages may also be obtained or enhanced if the mixture of liquid cell culture medium and first charged, particulate material is, prior to membrane filtration, mixed with second microsized charged, particulate material, e.g. ion exchange resin, bearing a charge opposite that of the first charged, particulate material.

For example, by the addition of microsized charged, particulate material to the liquid cell culture medium, prior to membrane filtration of the medium, the advantage of a 20% to several-fold increase in flow rate through a filtration membrane may be obtained, resulting from a substantial reduction in the formation of secondary layers of particles and/or solutes on the membrane surface (the concentration polarization phenomenon) which tend to gelatinize and to foul the membrane, and possibly also from a change in the membrane rejection coefficient. The commercial importance of such an advantage is readily apparent. A change in the membrane rejection coefficient may be obtained when the liquid cell culture medium contains protein, as the addition of microsized charged, particulate material, prior to membrane filtration of the medium, enables the rejection coefficient for given proteins relative to a specific membrane to be reduced and thereby allows passage, through a membrane, of proteins that, in the absence of the charged, particulate material, would be retained by the membrane.

According to the present invention there is provided a method of separating components of a liquid cell culture medium by membrane filtration, which comprises

(a) mixing together the medium and an effective amount of first charged, particulate material, e.g. ion exchange resin, to form a suspension of matter in the medium, the charged, particulate material having an average diameter in the range of from about 0.01 to about 2.5 micrometers, e.g. from about 0.01 to 1.5 micrometer, and bearing 0.1 to 1.5 functional groups per monomer unit; and

(b) recovering the suspended matter using membrane filtration.

In this specification, the expression "cell culture medium" means a fermentation broth (i.e., the liquid medium in which biological substances are grown), or filtrates or liquid fractions obtained from fermentation broths, including or excluding cells, cell debris and other material resulting from cell lysis, if practiced. The biological substances include plant, animal and microbial cells, genetically engineered cells, and products thereof.

The preferred charged, particulate materials useful in the method of the invention are fine particle size ion exchange resins such as described in the previously mentioned US-A-4,200,695 and US-A-4,537,683, which patents are incorporated herein by reference. Small charged particles of other types, functionality and/or exchange capacities may be used, such as conventional ion exchange resins produced by suspension polymerization or other technique, provided the resins have effective charge densities and particle size for use in the invention. The particle size of these resins may be reduced, as needed, by grinding, for example, in a known manner. Such resins are described, for example, in US-A-3,037,052; 3,637,535; 3,843,566; 3,791,866; 3,275,548; and 3,357,158, all of which are incorporated herein by reference. Other charged particles useful in the present invention include, but are not limited to, those particles having a diameter of 2.5 micrometers or smaller, which are insoluble in the liquid medium in which they are to be used and which have a charge at their surfaces that is available for interaction with other components in the liquid medium.

The ion exchange resins of US-A-4,200,695 and 4,537,683, be they positively or negatively charged, are

EP 0 244 999 B1

composed of crosslinked polymers in the shape of approximately spherical beads having diameters in the range of about 0.01 to 1.5 micrometers. One method by which resins having these properties can be prepared comprises emulsion polymerization of monomers, functionalizing the resulting coagulum, and then redispersing the resultant functionalized polymeric material. Useful ion exchange resins bear about 0.1 to 1.5 functional groups per monomer unit, which groups can be strongly acidic (e.g. $-SO_3H$ groups), weakly acidic (e.g. -COOH groups), strongly basic (e.g. quaternary ammonium groups), or weakly basic (e.g. tertiary amine groups).

Fermentation broths, produced by growing fungi, yeasts, bacteria or other cells of biological origin in conventional liquid culture media, may be filtered according to the method of the invention. After microbial growth and fermentation are finished, the broth will usually contain cells, cell debris, spent nutrients, biological products and various contaminants. The broths may themselves be filtered in accordance with the method of the invention or the solid material may first be removed by centrifugation, conventional filtration or other separation techniques and the invention practiced on the supernatant or any fraction thereof. The invention may also be practiced both on the fermentation broth and on any liquid portions or fractions thereof, including the supernatant.

The particular charged particles to be used in any given case is a matter of selection, based on well-known principles of microbiology and fermentation biochemistry in light of the charge and charge densities of solids and solutes in the cell culture media, and is easily made by one skilled in these arts. The terms "charged particle" and "charged particulate material", as used herein, are understood to include all particles bearing charges that are available for interaction with other components in a liquid medium, including charges limited to the particle surface; it includes both deliberately functionalized particles and those with naturally occurring charges, as well as both particles soluble in, and particles insoluble in, the liquid medium.

The method of this invention may be carried out by adding the first charged, particulate material of choice to the cell culture medium, e.g. fermentation broth, and then stirring the mixture until the suspension is homogeneous. In a preferred embodiment, a second charged, particulate material of choice, having a charge opposite that of the first material, is then added to the cell culture medium e.g. with stirring. The floc which forms in the medium will contain the charged particles, microbial cells (if present) and other medium components including particulate components and charged components, if present.

In a more preferred embodiment of the method of this invention, the first charged, particulate material is positively charged and immediately forms a floc with negatively charged components of the cell culture medium. The optional, subsequent addition of the negatively charged components of the cell culture medium. The optional, subsequent addition of the negatively charged material, stabilizes and increases the size of the previously formed floc by interacting with the positively charged material and other medium components.

The amounts of each of the charged particles ordinarily is in the range of about 0.01 to 0.5%, e.g. 0.05 to 0.5%, and preferably 0.1 to 0.25%, by volume, based on the volume of the cell culture medium.

After addition of the charged, particulate material(s) to the cell culture medium, the resulting suspension may then be passed through a membrane filter, e.g. in the usual manner. Covneniently, the membrane filters are those semipermeable membranes known in the art for their ability to remove dissolved or dispersed matter by ultrafiltration or microfiltration, but excluding separation of dissolved salts by a technique known as reverse osmosis.

In summary, the use, in accordance with the present invention, of microsized charged, particulate material(s), e.g. the use of first charged, particulate material and second charged, particulate material having a charge opposite that of the first charged, particulate material, enables the transmembrane flux rate of cell culture media to be vastly improved; the high flux rate remaining constant, in some cases, over at least a five-fold concentration of the medium. At the same time, when the medium contains proteins, enzymes and other products to be purified or recovered, the method of the invention enables decreased concentration polarization and reduced rejection of these products by the membrane filtration to be obtained, which results in a more complete separation and improved recovery of the products. These benefits depend on the formation of a floc upon addition of the first charged, particulate material; additional benefits depend on stabilization of the floc by the optional subsequent addition of the second charged, particulate material. The stabilization is believed to result from the linking of complexed cells (or other materials), positively charged particles, solutes and negatively charged particles through ionic interactions. It is believed that the floc adsorbs and removes from the liquid medium those components that are responsible for low flux rates in untreated media. Even in those cases in which the viscosity of the cell culture medium increases in the presence of the floc, the resultant decreases in flux rate are counteracted by decreases in formation of gel layers on the membrane caused by concentration polarization.

3

The present invention will now be further illustrated by way of the following examples which are for illustrative purposes only and are not to be construed as imposing any limitation on the scope of the invention. In these examples, all parts and percentages are by weight unless otherwise indicated.

The charged, particulate materials of the examples are beads prepared by emulsion polymerization as described in US-A-4,200,695 and 4,537,683 and are characterized as follows:

Resin A: Strong base, styrene/divinylbenzene/aminoalkyl methacrylate gellular copolymer, containing 5% divinylbenzene crosslinker, the tertiary amine functionality having been quaternized, and having an anion exchange capacity of 2.8 meq/g dry and an average particle diameter of 0.11 ± 0.02 micrometer, chloride form.

Resin B: Strong acid, sulfonic acid functionalized, styrene/divinylbenzene gellular copolymer, containing 7.3% divinylbenzene crosslinker, and having a cation exchange capacity of 5.1 meq/g dry and an average particle diameter of 0.26 ± 0.02 micrometer, hydrogen form.

Resin C: Strong base, quaternary amine functionalized, styrene/divinylbenzene gellular copolymer, containing 1.8% divinylbenzene crosslinker, and having an anion exchange capacity of 3.8 meq/g dry and an average particle diameter of 0.22 ± 0.02 micrometer.

Resin D: Strong base, quaternary amine functionalized, styrene/divinylbenzene macroreticular copolymer, containing 3% divinylbenzene crosslinker, and having an anion exchange capacity of 4.0 meq/g dry, and ground to an average particle size of 1.1 micrometer and a range of smaller than 0.4 micrometers to 5 micrometers.

Examples 1 and 2 describe the effects on flux rate and protein recovery of treatment of a fermentation broth with Resin A and Resin B, respectively, for comparison with Example 3 in which both of Resins A and B are used. Example 4 describes ultrafiltration of fermentation broths treated with both of the resins, and concentration by ultrafiltration of the separated, resuspended flocs. Example 5 shows flux rates and protein recovery when particle-free supernatants of the preparation of Example 4 are ultrafiltered with both of the resins. Example 6 is similar to Example 5 but shows the effects, on cell-free supernatants, of the ultrafiltration using both of the resins. Examples 7 and 8 show the effect on ultrafiltration and microfiltration flux rates, respectively, of a fermentation broth treated with Resin A. Example 9 shows the effect of other small anion exchange resins on microfiltration of a fermentation broth. Examples 10 and 11 show the effect of combined Resins A and B on ultrafiltration and microfiltration of a yeast cell suspension, while Example 12 shows the effect of combined Resins A and B on microfiltration of an albumen sample.

Example 1

Whole cells of Bacillus licheniformis, ATCC 21415, were grown on a liquid medium containing 3% starch, 1% glucose, 5% soybean meal hydrolysate, 1% ammonium phosphate, 0.03% potassium chloride and 0.02% magnesium sulfate at 30°C and pH 7.0 for four days. The final dry cell weight was 10 g/liter. At the end of cultivation, 1 liter of crude fermentation broth was concentrated by ultrafiltration at room temperature using a hollow fiber filtration module with porosity of 100,000 MW (Romicon HF-1-43 PM 100). The hollow fiber module consisted of membranes made from polysulfone. This module had twenty-five fibers with an internal diameter of 1.1 mm, a surface area of 0.093 $m^2$ (1 $ft^2$) and a length of 40 cm. The operating parameters used were a mean transmembrane pressure of 117 kPa (17 psi) and recirculation flow rate of 2 liters/min.

The average flux permeate rate was measured and compared with these obtained when a floc formed by the addition of Resin A was added to the fermentation broth. The initial extracellular protein, the protein adsorbed in the membrane and the protein rejection were also determined.

The average transmembrane filtrate flux rate in this and subsequent examples was calculated in a known manner, using Simpson's rule in the integration of the flux rate, $\bar{J}$, obtained over a filtration time corresponding to a 5- to 7-fold cell concentration:

$$\bar{J} = \frac{1}{t} \int_{0}^{tc} J\,dt$$

where

J     is the instantaneous flux rate,

t is time, and

tc is the time required to reach a specific concentration.

The results of the measurements are given in Table 1 from which it will be seen that improvement in flux rate was obtained over the control, the improvements increasing with Resin A concentration.

## Table 1

### Influence of microsized anion exchange resin on the ultrafiltration characteristics of a *B. licheniformis* broth.

| Resin A Concentration (%, wt/v) | Average Transmembrane Flux rate (liter/hr/m$^2$) |
| --- | --- |
| 0 (Control) | 12.5 |
| 0.05 | 19.6 |
| 0.10 | 26.9 |
| 0.25 | 31.5 |

### Example 2

The procedure of Example 1 was repeated in all essential respects except that Resin B was used in place of Resin A. No floc formed upon admixture of the broth with Resin B. The results are shown in Table 2 and indicate substantially lower efficacy than was achieved with Resin A in Example 1.

## Table 2

Influence of microsized cation resin on the ultrafiltration characteristics of a B. licheniformis broth.

| Resin B Concentration ($\%$, wt/v) | Average Transmembrane Flux rate (liter/hr/m$^2$) |
|---|---|
| 0 (Control) | 12.5 |
| 0.05 | 17.0 |
| 0.10 | 19.0 |
| 0.25 | 21.9 |

Example 3

The procedure of Example 1 was repeated in all essential respects except that the effect on the performance of the ultrafiltration of adding both Resin A and Resin B was studied. Table 3 shows the results as compared with the control (Table 1). It will be seen that, when comparing treatments with resins of the same concentrations, substantial increase in flux rate was obtained when the order was reversed. Resin A followed by Resin B represents a 24.7% increase in flux rate over that of the run in which Resin B was followed by Resin A.

The protein effectively rejected by the membrane is defined as the total amount of protein present in the concentrate fraction plus the total protein in the concentration polarized gel layer associated with the membrane. The protein in the gel layer (P) is obtained by difference between the total protein present in the cleared supernatant of the starting broth and the sum of the protein in the filtrate (permeate) and the cleared concentrate after filtration. Protein rejection in this and subsequent examples is expressed as the percentage of the starting protein in solution that was not recoverable in the filtrate; the calculation is:

$$\text{Average Protein Rejection} = 1 - \frac{\ln\left(1 - \frac{P_f V_f}{P_i V_i}\right)}{\ln\left(1 - \frac{V_f}{V_i}\right)}$$

where

$P_f$ = the protein, in milligrams, in the filtrate fraction

$V_f$ = the volume, in milliliters, of the filtrate fraction

$P_i$ = the initial protein in solution (mg)
$V_i$ = the initial solution volume (ml).

## Table 3

Influence of order of addition and amounts of Resins A and B on ultrafiltration characteristics of a B. licheniformis broth.

| Resin Added First | Added Second | Flux (liter/hr/m²) | Protein Rejection | Enzyme Rejection |
|---|---|---|---|---|
| none | none | 12.5 | 0.91 | 0.18 |
| Resin B (0.10%) | Resin A (0.10%) | 20.3 | 0.89 | 0.08 |
| Resin B (0.10%) | Resin A (0.25%) | 29.5 | 0.89 | 0.41 |
| Resin B (0.25%) | Resin A (0.10%) | 29.8 | 0.88 | 0.14 |
| Resin A (0.25%) | Resin B (0.10%) | 36.9 | 0.76 | 0.09 |

Example 4

(A). Cell cultivation was carried out for 48 hours essentially as described in Example 1. The resulting dry cell weight was 4 g/liter. To 1 liter of the fermentation broth were added Resin A and then Resin B to final 0.25% and 0.10% concentrations, respectively. The control sample was 1 liter of the fermentation broth.

(B). B. lichenformis cells and flocculated structures, containing mixed resins, whole cells and components of the medium, were separated by centrifugation at 7,000 x g for 10 minutes from the control and the treated sample of (A) above. The particulate material, whole cells or flocs were washed with 1 liter of distilled water, centrifuged for 10 minutes and resuspended in distilled water at the same volume as the initial samples (1 liter). The water-suspended whole cells (control) and the water-suspended flocs were concentrated by ultrafiltration, using the same cartridge as described in Example 1. Average flux rates are shown in Table 4. It will be noted that the combination of Resins A and B greatly increased the flux rate.

## Table 4

Influence of mixed ion exchange resin flocs on flux of B. licheniformis water suspensions.

| Sample Treatment | Average Transmembrane Flux Rate (liter/hr/m$^2$) |
|---|---|
| Control, no additions (washed cells) | 77 |
| Resin A (0.25%) + Resin B (0.1%) (washed flocs) | 104 |

Example 5

The procedure of Example 4 was repeated in all essential respects except that the performances of the ultrafiltration of the supernatants of the preparations described in Example 4 were measured (Table 5). The substantial improvements afforded by the combined resins are readily apparent.

## Table 5

Ultrafiltration of particle-free supernatants of crude and resin-treated whole cell broth.

| Sample Treatment (%, wt/v) | Average Transmembrane Flux Rate (liter/hr/m$^2$) |
|---|---|
| Control | 8.13 |
| Resin A (0.25%) Resin B (0.10%) | 28.5 |

Example 6

The procedure of Example 5 was repeated in all essential respects except that Resin A and then Resin

8

B were added to cell-free supernatant at final concentrations of 0.25% and 0.10%, respectively. The improved performance of the ultrafiltration of the ion exchanger-treated, cell-free supernatant is shown in Table 6.

## Table 6

Ultrafiltration of cell-free supernatant treated with mixed resins.

| Sample Treatment (%, wt/v) | Average Transmembrane Flux Rate $(\text{liter/hr/m}^2)$ |
|---|---|
| Control (cell-free supernatant) | 8.13 |
| Resin A (0.25%) Resin B (0.10%) | 22.8 |

Example 7

Whole cells of Bacillus sp., ATCC 21536, were grown on a liquid medium containing 2% starch, 0.5% yeast extract, 0.5% peptone, 0.1% monopotassium phosphate, 0.02% magnesium sulfate and 1% sodium carbonate at 37°C and pH 10.0 for three days. The final dry cell concentration, determined by precipitating the cells in a broth sample, washing the resulting cell pellet, drying it at 80°C for 30 hours and weighing it, was 4.6 g/liter. At the end of cultivation, one liter of crude fermentation broth was concentrated by ultrafiltration as described in Examples 1 and 3. The resins used and results are given in Table 7, and show that a substantial improvement (41%) in flux rate was obtained when Resin A was added to the fermentation medium. The flux was increased 59.3% by adding Resin B to the floc previously formed with Resin A.

## Table 7

| Charged Particle Preparation (%, wt/v) | Average Transmembrane Flux Rate $(\text{liter/hr/m}^2)$ | Average Protein Rejection |
|---|---|---|
| None | 14.1 | 0.94 |
| Resin A (0.1%) | 19.9 | 0.93 |
| Resin A (0.1%) + Resin B (0.04%) | 31.7 | 0.85 |

Example 8

The procedure of Example 7 was repeated except that the whole fermentation broth was concentrated by microfiltration at room temperature using a hollow fiber filtration module with a porosity of 0.1 μm (Romicon HF-1-47MP). This module has twenty-five fibers with an internal diameter of 1.1 mm, a surface area of 930 cm$^2$ and a length of 40 cm. The average permeate flux rate was measured and compared with those obtained when Resin A was added to the Bacillus sp. fermentation broth. The results of the measurements are given in Table 8, from which it may be seen that with a resin concentration of 0.1% wt/v, an improvement of 55% in flux rate was obtained over the control.

### Table 8

| Resin A Concentration (%, wt/v) | Average Transmembrane Flux Rate (liter/hr/m$^2$) |
|---|---|
| None | 15.4 |
| 0.05 | 28.7 |
| 0.10 | 23.9 |

Example 9

The procedure of Example 8 was repeated except that other microsized anion exchange resins were used for the concentration of a 4-day-old Bacillus broth. The average flux rates obtained during microfiltration are shown in Table 9 and indicate substantial improvements may be obtained by the addition of the microsized resins useful in the present invention.

### Table 9

| Charged Particle Concentration (%, wt/v) | Average Resin Diameter (μm) | Average Flux Rate (liter/hr/m$^2$) |
|---|---|---|
| None | | 14.2 |
| Resin A (0.10%) | 0.12 | 31.9 |
| Resin C (0.10%) | 0.22 | 32.7 |
| Resin D (0.18%) | 1.0 | 26.2 |

Example 10

Whole yeast cells of Saccharomyces cerevisiae (5 g/liter) were suspended in 0.15 M sodium chloride at pH 7.0. Two 4-liter portions were treated with the resins useful in the present invention by first adding the amount of Resin A indicated in Table 10, and subsequently adding the indicated amount of Resin B. The treated portions and untreated suspension were concentrated by ultrafiltration as described in Example 1

10

and the results are given in Table 10. The resin additions increased the flux rates by 53% and 82%.

## Table 10

| Charged Particle Preparation (% wt/v) | Average Flux Rate (liter/hr/m$^2$) |
|---|---|
| None | 38.4 |
| Resin A (0.001%) + Resin B (0.0004%) | 58.8 |
| Resin A (0.005%) + Resin B (0.002%) | 69.9 |

Example 11

The procedure of Example 10 was repeated except that the yeast cell suspension was concentrated by microfiltration as described in Example 8. Table 11 shows the effect of Resin A and mixed Resins A and B on the average flux rates.

## Table 11

| Charged Particle Preparation (% wt/v) | Average Flux Rate (liter/hr/m$^2$) |
|---|---|
| None | 35.0 |
| Resin A (0.005%) | 50.5 |
| Resin A (0.005%) + Resin B (0.002%) | 57.0 |

Example 12

The procedure of Example 11 was repeated except that a 0.5 g/liter bovine serum albumen (BSA) solution was added to the yeast cell suspension and the protein rejection was measured. Table 12 shows the ability of mixed charge Resins A and B to improve flux and protein rejection.

## Table 12

| Charged Particle Preparation (wt/v) | Average Flux Rate $(\text{liter/hr/m}^2)$ | Average Protein Rejection |
|---|---|---|
| None | 45.5 | 0.75 |
| Resin A (0.001%) + Resin B (0.0004%) | 66.1 | 0.42 |

**Claims**

1. A method of separating components of a liquid cell culture medium by membrane filtration, which comprises:

   (a) mixing together the medium and an effective amount of first charged, particulate material to form a suspension of matter in the medium, the charged, particulate material having an average diameter of from about 0.01 to about 2.5 micrometers and bearing 0.1 to 1.5 functional groups per monomer unit; and

   (b) recovering the suspended matter using membrane filtration.

2. A method as claimed claim 1, wherein the liquid cell culture medium comprises fermentation broth, for example, broth of a bacterium or of a yeast.

3. A method as claimed in claim 1 or claim 2, wherein the first charged, particulate material comprises ion exchange resin, e.g. anion exchange resin.

4. A method as claimed in any preceding claim, wherein, prior to step (b), the suspension formed in stap (a) and an effective amount of second charged particulate material, e.g. ion exchange resin, bearing a charge opposite to that of the first charged, particulate material, bearing 0.1 to 1.5 functional groups per monomer unit, and having an average diameter of from about 0.01 to about 2.5 micrometers, are mixed together.

5. A method as claimed in claim 4, wherein the first charged, particulate material comprises anion exchange resin and the second charged, particulate material comprises cation exchange resin.

6. A method as claimed in claim 5, wherein the first and second charged, particulate materials are each present in an amount of about 0.01 to 0.5, for example, about 0.05 to 0.5%, by volume, based on the volume of the cell culture medium.

7. A method as claimed in claim 4, wherein the first charged particulate material comprises strongly basic resin and the second charged particulate material comprises strongly acidic resin.

8. A method as claimed in any of claims 4 to 7, wherein solid components of the medium, subsequent to mixing of the liquid medium with the first and second charged, particulate material, are separated from the medium and resuspended in a second, aqueous, liquid medium, which is subequently subjected to membrane filtration.

9. A method as claimed in any of claims 4 to 7, wherein solid components of the medium, subsequent to mixing of the liquid medium with the first and second charged, particulate materials, are separated from the medium and the medium is subsequently subjected to membrane filtration.

10. A method as claimed in any preceding claim, wherein the first particulate material has an average diameter of from about 0.01 to 1.5 micrometers.

11. A method as claimed in any of claims 4 to 10, wherein the second particulate material has an average diameter of from about 0.01 to 1.5 micrometers.

12. Use of charged, particulate material for aiding the separation of components of a liquid cell culture medium by membrane filtration, characterized in that:-
    (a) the medium and an effective amount of first charged, particulate material, for example ion exchange resin, are mixed together to form a suspension of matter in the medium, the charged, particulate material having an average diameter of from about 0.01 to about 2.5 micrometers and bearing 0.1 to 1.5 functional groups per monomer unit; and
    (b) the suspended matter is recovered using membrane filtration.

**Patentansprüche**

1. Verfahren zur Abtrennung von Komponenten aus einem flüssigen Zellkulturmedium durch Membranfiltration, bei dem
    (a) das Medium mit einer wirksamen Menge eines ersten geladenen, partikulären Materials zur Ausbildung einer Stoffsuspension in dem Medium gemischt wird, wobei das geladene, partikuläre Material einen durchschnittlichen Durchmesser von etwa 0,01 bis etwa 2,5 $\mu$m hat und 0,1 bis 1,5 funktionelle Reste pro Monomereinheit aufweist, und
    (b) der suspendierte Stoff unter Verwendung einer Membranfiltration erhalten wird.

2. Verfahren nach Anspruch 1, worin das flüssige Zellkulturmedium eine Fermentationsbrühe, beispielsweise eine Brühe eines Bakteriums oder einer Hefe, umfaßt.

3. Verfahren nach Anspruch 1 oder 2, worin das erste geladene, partikuläre Material ein Ionenaustauscherharz, beispielsweise ein Anionenaustauscherharz, umfaßt.

4. Verfahren nach einem der vorhergehenden Ansprüche, worin vor Schritt (b) die in Schritt (a) gebildete Suspension mit einer wirksamen Menge eines zweiten geladenen, partikulären Materials, beispielsweise eines Ionenaustauscherharzes, das eine zu der des ersten geladenen, partikulären Materials entgegengesetzte Ladung aufweist, 0,1 bis 1,5 funktionelle Reste pro Monomereinheit aufweist und einen durchschnittlichen Durchmesser von etwa 0,01 bis etwa 2,5 $\mu$m hat, gemischt wird.

5. Verfahren nach Anspruch 4, worin das erste geladene, partikuläre Material ein Anionenaustauscherharz umfaßt und das zweite geladene, partikuläre Material ein Kationenaustauscherharz umfaßt.

6. Verfahren nach Anspruch 5, worin das erste und zweite geladene, partikuläre Material jeweils in einer Menge von etwa 0,01 bis 0,5, beispielsweise etwa 0,05 bis 0,5 Vol.-%, basierend auf dem Volumen des Zellkulturmediums, vorliegt.

7. Verfahren nach Anspruch 4, worin das erste geladene, partikuläre Material ein stark basisches Harz umfaßt und das zweite geladene, partikuläre Material ein stark saures Harz umfaßt.

8. Verfahren nach einem der Ansprüche 4 bis 7, worin die festen Komponenten des Mediums nach Mischung des flüssigen Mediums mit dem ersten und zweiten geladenen, partikulären Material von dem Medium abgetrennt und in einem zweiten wäßrigen, flüssigen Medium, das nachfolgend einer Membranfiltration unterzogen wird, resuspendiert werden.

9. Verfahren nach einem der Ansprüche 4 bis 7, worin die festen Komponenten des Mediums nach Mischung des flüssigen Mediums mit dem ersten und zweiten geladenen, partikulären Material von dem Medium abgetrennt werden und das Medium nachfolgend einer Membranfiltration unterzogen wird.

**10.** Verfahren nach einem der vorhergehenden Ansprüche, worin das erste partikuläre Material einen durchschnittlichen Durchmesser von etwa 0,01 bis 1,5 μm hat.

**11.** Verfahren nach einem der Ansprüche 4 bis 10, worin das zweite partikuläre Material einen durchschnittlichen Durchmesser von etwa 0,01 bis 1,5 μm hat.

**12.** Verwendung eines geladenen, partikulären Materials zur Unterstützung der Abtrennung von Komponenten aus einem flüssigen Zellkulturmedium durch Membranfiltration,
**dadurch gekennzeichnet,** daß
(a) das Medium mit einer wirksamen Menge eines ersten geladenen, partikulären Materials, beispielsweise eines Ionenaustauscherharzes, zur Ausbildung einer Stoffsuspension in dem Medium gemischt wird, wobei das geladene, partikuläre Material einen durchschnittlichen Durchmesser von etwa 0,01 bis etwa 2,5 μm hat und 0,1 bis 1,5 funktionelle Reste pro Monomereinheit aufweist, und
(b) der suspendierte Stoff unter Verwendung einer Membranfiltration erhalten wird.

**Revendications**

**1.** Procédé de séparation de composants d'un milieu liquide de culture de cellules, par filtration sur membrane, comprenant:
(a) le mélangeage l'un avec l'autre du milieu et d'une quantité efficace d'un premier matériau particulaire chargé pour la formation d'une suspension de matière dans le milieu, le matériau particulaire chargé ayant un diamètre moyen de particules allant d'environ 0,01 à environ 2,5 μm et portant de 0,1 à 1,5 groupes fonctionnels par motif monomère; et
(b) la récupération de la matière en suspension au moyen de filtration sur membrane.

**2.** Procédé selon la revendication 1, dans lequel le milieu liquide de culture de cellules comprend un bouillon de fermentation, par exemple un bouillon de culture d'une bactérie ou d'une levure.

**3.** Procédé selon la revendication 1 ou 2, dans lequel le premier matériau particulaire chargé comprend une résine échangeuse d'ions, par exemple une résine échangeuse d'anions.

**4.** Procédé selon l'une quelconque des revendications précédentes, dans lequel, avant l'étape (b), on mélange l'une avec l'autre la suspension formée dans l'étape (a) et une quantité efficace d'un second matériau particulaire chargé, par exemple une résine échangeuse d'ions, portant une charge opposée à celle du premier matériau particulaire chargé, portant de 0,1 à 1,5 groupes fonctionnels par motif monomère et ayant un diamètre moyen de particules allant d'environ 0,01 à environ 2,5 μm.

**5.** Procédé selon la revendication 4, dans lequel le premier matériau particulaire chargé comprend une résine échangeuse d'anions et le second matériau particulaire chargé comprend une résine échangeuse de cations.

**6.** Procédé selon la revendication 5, dans lequel le premier et le second matériaux particulaires chargés sont présents chacun en une quantité allant d'environ 0,01 à 0,5, par exemple d'environ 0,05 à 0,5 %, en volume, par rapport au volume du milieu de culture de cellules.

**7.** Procédé selon la revendication 4, dans lequel le premier matériau particulaire chargé comprend une résine fortement basique, et le second matériau particulaire chargé comprend une résine fortement acide.

**8.** Procédé selon l'une quelconque des revendications 4 à 7, dans lequel les composants solides du milieu, après mélangeage du milieu liquide avec le premier et le second matériaux particulaires chargés, sont séparés du milieu et remis en suspension dans un second milieu liquide aqueux qui est ensuite soumis à une filtration sur membrane.

**9.** Procédé selon l'une quelconque des revendications 4 à 7, dans lequel les composants solides du milieu, après mélangeage du milieu liquide avec le premier et le second matériaux particulaires chargés, sont séparés du milieu et le milieu est ensuite soumis à une filtration sur membrane.

**10.** Procédé selon l'une quelconque des revendications précédentes, dans lequel le premier matériau particulaire a un diamètre moyen de particules allant d'environ 0,01 à 1,5 $\mu$m.

**11.** Procédé selon l'une quelconque des revendications 4 à 10, dans lequel le second matériau particulaire a un diamètre moyen de particules allant d'environ 0,01 à 1,5 $\mu$m.

**12.** Utilisation d'un matériau particulaire chargé, pour faciliter la séparation de composants d'un milieu liquide de culture de cellules, par filtration sur membrane, caractérisée en ce que:

(a) on mélange l'un avec l'autre le milieu et une quantité efficace d'un premier matériau particulaire chargé, par exemple une résine échangeuse d'ions, pour la formation d'une suspension de matière dans le milieu, le matériau particulaire chargé ayant un diamètre moyen de particules allant d'environ 0,01 à environ 2,5 $\mu$m et portant de 0,1 à 1,5 groupes fonctionnels par motif monomère; et
(b) on récupère la matière en suspension au moyen de filtration sur membrane.